(19) Europäisches Patentamt
European Patent Office
Office européen des brevets

(11) Veröffentlichungsnummer : **0 011 714**
**B1**

(12) **EUROPÄISCHE PATENTSCHRIFT**

(45) Veröffentlichungstag der Patentschrift : **21.10.81**

(51) Int. Cl.³ : **C 07 F 7/08**, C 08 G 77/38, A 61 C 9/00, B 01 J 31/30

(21) Anmeldenummer : **79104155.1**

(22) Anmeldetag : **26.10.79**

(54) **Verfahren zum Anlagern von Si-H-Verbindungen an aliphatische Mehrfachbindung.**

(30) Priorität : **26.10.78 DE 2846621**

(43) Veröffentlichungstag der Anmeldung : **11.06.80 (Patentblatt 80/12)**

(45) Bekanntmachung des Hinweises auf die Patenterteilung : **21.10.81 Patentblatt 81/42**

(84) Benannte Vertragsstaaten : **AT BE CH DE FR GB IT NL SE**

(56) Entgegenhaltungen :

**DE - A1 - 2 724 822**
**US - A - 3 159 601**
**US - A - 3 159 662**
**US - A - 3 453 234**
**US - A - 3 814 730**

(73) Patentinhaber : **WACKER-CHEMIE GMBH**
**Prinzregentenstrasse 22**
**D-8000 München 22 (DE)**

(72) Erfinder : **Kreis, Gerhard, Dr. Dipl.-Chem.**
**Springerstrasse 7**
**D-8000 München 71 (DE)**
Erfinder : **Wegehaupt, Karl-Heinrich, Dr. Dipl.-Chem.**
**Marktler Strasse 78**
**D-8263 Burghausen (DE)**

**EP 0 011 714 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 17, rue du Louvre, 75001 Paris, France

Verfahren zum Anlagern von Si-H-Verbindungen an aliphatische Mehrfachbindung

Es ist bekannt, die Anlagerung von Si-gebundenem Wasserstoff an aliphatische Mehrfachbindung, eine Anlagerung, die auch als « Hydrosilylierung » bezeichnet wird, durch Katalysatoren, insbesondere Platinkatalysatoren, zu fördern. Hierzu sei z.B. auf die US-PS 38 14 730 (« Karstedt »-Katalysatoren), der die FR-PS 15 48 775 entspricht, verwiesen.

Gegenüber den bisher bekannten Katalysatoren für die Anlagerung von Si-gebundenem Wasserstoff an aliphatische Mehrfachbindung haben die erfindungsgemäß verwendeten Platinkomplexe den Vorteil, daß sie noch wirksamer sind und/oder leichter durch Fällungsreaktionen in reiner Form hergestellt und damit auch leichter dosiert werden können.

Gegenstand der Erfindung ist ein Verfahren zum Anlagern von Si-gebundenem Wasserstoff an aliphatische Mehrfachbindung in Gegenwart von Platinkatalysator, dadurch gekennzeichnet, daß als Platinkatalysator mindestens ein Platinkomplex aus der Gruppe von Verbindungen der allgemeinen Formel

$$(Dien)\ PtX_2,$$

wobei Dien einen cyclischen, Kohlenwasserstoff mit 2 aliphatischen Kohlenstoff-Kohlenstoff-Doppelbindungen und X gleiche oder verschiedene Halogenatome und/oder gleiche oder verschiedene Alkylreste bedeutet, und Verbindungen der allgemeinen Formel

$$(R_2SO)\ (Z)\ PtY_2,$$

wobei R gleiche oder verschiedene, gegebenenfalls substituierte Kohlenwasserstoffreste, Z einen Kohlenwasserstoff mit einer Kohlenstoff-Kohlenstoff-Doppelbindung und Y gleiche oder verschiedene Halogenatome bedeutet, verwendet wird.

Beispiele für cyclische, 2 aliphatische Kohlenstoff-Kohlenstoff-Doppelbindungen aufweisende Kohlenwasserstoffe Dien sind Dicyclopentadien und 1,5-Cyclooktadien.

Bei Halogenatomen X kann es sich um Fluor, Chlor, Brom oder Jod handeln. Chlor ist bevorzugt.

Alkylreste X enthalten vorzugsweise 1 bis 4 Kohlenstoffatome, wie der Methyl-, Äthyl-, Propyl-, Isopropyl-, n-Butyl-, sec.-Butyl- und tert.-Butylrest.

Wegen seiner hohen Wirksamkeit bereits bei Raumtemperatur ist als Platinkomplex der allgemeinen Formel

$$(Dien)\ PtX_2$$

Dicyclopentadien-platindichlorid, also die Verbindung der Formel

$$(C_{10}H_{12})\ PtCl_2,$$

besonders bevorzugt. Weitere Beispiele für Platinkomplexe der allgemeinen Formel

$$(Dien)\ PtX_2$$

sind Verbindungen der Formeln

$(C_{10}H_{12})PtBr_2$
$(C_{10}H_{12})PtJ_2$
$(C_8H_{12})PtCl_2$
$(C_8H_{12})PtBr_2$
$(C_8H_{12})Pt(CH_3)Cl$
$(C_8H_{12})Pt(CH_3)_2$
$(C_8H_{12})Pt(C_2H_5)_2$
$(C_{10}H_{12})Pt(CH_3)_2$
$(C_{10}H_{12})Pt(C_2H_5)_2$
$(C_{10}H_{12})Pt(C_4H_9)_2$.

In den vorstehenden Formeln bedeutet « $C_{10}H_{12}$ » jeweils Dicyclopentadien und « $C_8H_{12}$ » jeweils 1,5-Cyclooktadien.

Neben Dicyclopentadien-platindichlorid sind Dicyclopentadienplatin-$C_1$ bis $C_4$-alkyle,

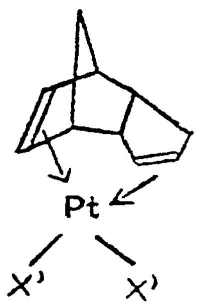

wobei X' ein Alkylrest mit 1 bis 4 Kohlenstoffatomen ist, bevorzugt.

Platinkomplexe der allgemeinen Formel

$$(Dien)\ PtX_2$$

und ihre Herstellung sind bekannt. Hierzu wird auf J. Chatt und Mitarbeiter, Journal of the Chemical Society, 1957, Seite 2 496 bis 2 505, und H. C. Clark und Mitarbeiter, Seite 411 bis 428, verwiesen.

Dicyclopentadien-platindichlorid kann nicht nur durch Umsetzung von $Na_2PtCl_4 \cdot 4H_2O$ mit Dicyclopentadien in n-Propanol, sondern auch durch Umsetzung von $K_2PtCl_4$ mit Dicyclopentadien in etwa 50 gewichtsprozentiger wäßriger Essigsäure erhalten werden. Aus Dicyclopentadien-platindichlorid kann durch Umsetzung mit Lithiumbromid in Aceton Dicyclopentadien-platindibromid und durch Umsetzung mit Natriumjodid in Aceton Dicyclopentadien-platindijodid hergestellt werden.

Die gegebenenfalls substituierten Kohlenwasserstoffreste R können aliphatisch oder aromatisch sein. Vorzugsweise enthalten sie

1 bis 10 Kohlenstoffatome. Die oben angegebenen Beispiele für Alkylreste X gelten auch für die Kohlenwasserstoffreste R. Weitere Beispiele für Kohlenwasserstoffreste R sind der Phenyl- und Benzylrest. Beispiele für substituierte Kohlenwasserstoffreste R sind der 2-Chloräthylrest, der Rest der Formel $-CH_2COOH$, der o-Chlorphenylrest, der c-Carboxyphenyl- und der o-Nitrophenylrest. Die Reste R können miteinander zu einem Ring verbunden sein, wie im Tetramethylenrest.

Die eine Kohlenstoff-Kohlenstoff-Doppelbindung aufweisenden Kohlenwasserstoffe Z können linear, verzweigt oder cyclisch sein. Vorzugsweise enthalten sie 2 bis 10 Kohlenstoffatome. Beispiele für Kohlenwasserstoffe Z sind Äthylen, Propylen, n-1-Buten, n-1-Penten, Cyclopenten, Styrol, cis-2-Hexen und 1-Nonen.

Bei den Halogenatomen Y kann es sich um Fluor, Chlor, Brom oder Jpd handeln. Chlor und Brom sind bevorzugt.

Ebenfalls wegen seiner hohen Wirksamkeit bereits bei Raumtemperatur ist als Platinkomplex der allgemeinen Formel

$$(R_2SO)\ (Z)\ PtY_2$$

Dimethylsulfoxyd-äthylen-platin-(II)-dichlorid, also die Verbindung der Formel

$$[(CH_3)_2SO]\ (C_2H_4)\ PtCl_2,$$

bevorzugt. Weitere Beispiele für Platinkomplexe der allgemeinen Formel

$$(R_2SO)\ (Z)\ PtY_2$$

sind Verbindungen der Formeln

$[(CH_3)_2SO]\ (C_3H_6)\ PtCl_2$
$[(CH_3)_2SO]\ (C_4H_8)\ PtCl_2$
$[(CH_3)_2SO]\ (C_5H_{10})\ PtCl_2$
$[(CH_3)_2SO]\ (C_5H_8)\ PtCl_2$
$[(CH_3)_2SO]\ (C_6H_5CH{=}CH_2)\ PtCl_2$
$[(CH_3)_2SO]\ (C_2H_4)\ PtBr_2$
$[(CH_3)_2SO]\ (C_3H_6)\ PtBr_2$
$[(C_6H_5)\ (CH_3)\ SO]\ (C_2H_4)\ PtCl_2$
$[(C_6H_5)\ (CH_3)\ SO]\ (C_3H_6)\ PtCl_2$
$[(C_6H_5)\ (CH_3)\ SO]\ (C_6H_5CH{=}CH_2)\ PtCl_2$
$[(C_6H_5)\ (CH_3)\ SO]\ (C_2H_4)\ PtBr_2$.

In den vorstehenden Formeln bedeutet « $C_4H_8$ » n-1-Buten, und « $C_5H_{10}$ » bedeutet n-1-Penten.

Platinkomplexe der allgemeinen Formel

$$(R_2SO)\ (Z)\ PtY_2$$

und ihre Herstellung sind bekannt. Hierzu wird auf H. Boucher und Mitarbeiter, Journal of the American Chemical Society, Bd. 99, 1977, Seite 6 253 bis 6 261, verwiesen.

In den Sulfoxyd-äthylen-platin-II-dihalogeniden läßt sich das Äthylen durch höher siedende, eine Kohlenstoff-Kohlenstoff-Doppelbindung aufweisende Kohlenwasserstoffe verdrängen.

Die im Rahmen des erfindungsgemäßen Verfahrens, das auch als « Verfahren zum Anlagern von Siliciumverbindung mit Si-gebundenem Wasserstoff an Verbindung mit aliphatischer Mehrfachbindung » bezeichnet werden kann, angewendeten Mengen an Katalysator können die gleichen sein wie bei den bisher bekannten Verfahren zum Anlagern von Si-gebundenem Wasserstoff an aliphatische Mehrfachbindung in Gegenwart von Platinkatalysator. Es sind dies vorzugsweise mindestens $10^{-10}$ Grammatom, insbesondere $10^{-8}$ bis $10^{-3}$ Grammatom, Platin, jeweils berechnet als elementares Platin, je Grammatom Si-gebundenen Wasserstoffs.

Die im Rahmen des erfindungsgemäßen Verfahrens angewandten Temperaturen und Drücke können ebenfalls die gleichen sein wie bei den bisher bekannten Verfahren zum Anlagern von Si-gebundenem Wasserstoff an aliphatische Mehrfachbindung in Gegenwart von Platinkatalysator. Es sind dies vorzugsweise Raumtemperatur bis 150 °C und der Druck der umgebenden Atmosphäre, also 1 bar oder etwa 1 bar. Falls erwünscht, können jedoch auch höhere oder niedrigere Drücke angewendet werden.

Das erfindungsgemäße Verfahren kann überall dort angewendet werden, wo monomere oder polymere Siliciumverbindungen mit Si-gebundenem Wasserstoff an monomere oder polymere Verbindungen mit aliphatischer Mehrfachbindung angelagert werden sollen. Bei dieser Anlagerung können je nach Wahl der aneinander anzulagernden Verbindungen andere monomere Siliciumverbindungen oder dimere bzw. polymere, Silicium enthaltende Verbindungen entstehen oder modifiziert werden.

Beispiele für die Herstellung von monomeren Siliciumverbindungen, für die das erfindungsgemäße Verfahren angewendet werden kann, sind die Herstellung von 3-Chlorpropyltrichlorsilan durch Umsetzung von Trichlorsilan mit Allylchlorid, die Herstellung von 3-Chlorpropylmethyldichlorsilan durch Umsetzung von Methyldichlorsilan mit Allylchlorid, die Herstellung von n-Propyltrichlorsilan durch Umsetzung von Propen mit Trichlorsilan, die Herstellung von Methacryloxypropyltrichlorsilan durch Umsetzung von Allylmethacrylat mit Trichlorsilan und die Herstellung von Vinylmethyldichlorsilan durch Umsetzung von Acetylen mit Methyldichlorsilan.

Beispiele für die Herstellung von dimeren bzw. polymeren, Silicium enthaltenden Verbindungen, für die das erfindungsgemäße Verfahren angewendet werden kann, sind die Umsetzung von Vinyltrichlorsilan mit Trichlorsilan zu Bis-(1,2-trichlorsilyl)-äthan, die Herstellung von Organosiloxanen mit SiC-gebundenen Estergruppen durch Anlagern von mindestens einem Diester der Allylbernsteinsäure an Organosiloxan mit Si-gebundenem Wasserstoff oder die Verminderung der Anzahl von aliphatischen Mehrfachbindungen in Polymeren, z.B. Poly-(oxyalkylen)-polyolen, durch Umsetzung von aliphatische

Mehrfachbindungen enthaltenden Polymeren dieser Art mit Organopolysiloxanen, die je Molekül mindestens zwei Si-gebundene Wasserstoffatome enthalten.

Zu der Modifizierung von polymeren, Silicium enthaltenden Verbindungen, für die das erfindungsgemäße Verfahren angewendet werden kann, gehört auch und insbesondere die Vernetzung, also Härtung oder Vulkanisation, von Massen auf Grundlage von Alkenylgruppen, insbesondere Vinylgruppen, und Si-gebundenen Wasserstoff enthaltenden Organopolysiloxanen. Bei dieser Vernetzung kann hohe Wirksamkeit des Katalysators bei Raumtemperatur, um einen raschen Ablauf der Vernetzung bei Raumtemperatur bzw. bei Temperaturen im Bereich von 18 bis 35 °C zu erzielen, von besonders großer Bedeutung sein. Deshalb wird das erfindungsgemäße Verfahren bevorzugt zur Vernetzung solcher Massen, bei denen es sich z.B. um Einbettungsmassen für elektrische oder elektronische Vorrichtungen oder Beschichtungsmassen, einschließlich Massen zum Herstellen von klebrige Stoffe abweisenden Überzügen, beispielsweise auf Papier, oder um Abformmassen, beispielsweise für die Herstellung von Formkörpern aus Beton, insbesondere aber zum Erzeugen von Abdrücken von menschlichen oder tierischen Zähnen, handeln kann, angewandt.

Selbstverständlich können auch bei dem erfindungsgemäßen Verfahren die Anlagerung von Si-gebundenem Wasserstoff an aliphatische Mehrfachbindung bei Raumtemperatur verzögernde Mittel, wie Benzotriazol, 1,3-Divinyl-1,1,3,3-tetramethyldisiloxan und/oder 2-Methyl-3-butin-2-ol, erforderlichenfalls mitverwendet werden.

In den folgenden Beispielen und Vergleichsversuchen beziehen sich alle Angaben von Teilen und Prozentsätzen auf das Gewicht, soweit nichts anderes angegeben ist.

a) Das in den folgenden Beispielen verwendete Gemisch aus Dicyclopentadien-platindichlorid und Verdünnungsmittel wurde hergestellt wie folgt:

0,2 g Dicyclopentadien-platindichlorid werden in 20 ml Methylenchlorid gelöst. Diese Lösung wird mit 100 g eines Vinyldimethylsiloxygruppen als endständige Einheiten aufweisenden Dimethylpolysiloxans mit einer Viskosität von 1 000 mPa.s bei 23 °C vermischt. Die so erhaltene Mischung wird bei 1 mbar und Raumtemperatur so lange gerührt, bis das Methylenchlorid verdunstet ist und enthält dann 0,1 % Platin, berechnet als Element.

b) Das in den folgenden Beispielen verwendete Gemisch aus Dimethylsulfoxyd-äthylen-platin-(II)-dichlorid und Verdünnungsmittel wurde hergestellt nach der vorstehend unter a) beschriebenen Arbeitsweise mit der Abänderung, daß 0,2 g Dimethylsulfoxyd-äthylen-platin-(II)-dichlorid anstelle der 0,2 g Dicyclopentadien-platindichlorid eingesetzt wurden. Die so erhaltene Mischung enthält ebenfalls 0,1 % Platin, berechnet als Element.

Beispiel 1

90 g des Vinyldimethylsiloxygruppen als endständige Einheiten aufweisenden Dimethylpolysiloxans mit einer Viskosität von 1 000 mPa.s bei 23 °C werden mit 2 g des Gemisches aus Platinkomplex und Verdünnungsmittel, dessen Herstellung oben unter a) beschrieben wurde, vermischt. Das so erhaltene Gemisch wird 5 Tage bei Raumtemperatur gelagert. Dann werden in dieses Gemisch 10 g eines Mischpolymerisates aus Dimethylsiloxan-, Methylhydrogensiloxan- und Trimethylsiloxaneinheiten, wobei das Molverhältnis der Dimethylsiloxaneinheiten zu den Methylhydrogensiloxaneinheiten 1 : 9 beträgt und das Mischpolymerisat eine Viskosität von 700 mPa.s bei 23 °C hat, eingerührt. Die Zeit zwischen dem Beginn dieses Einrührens und merklicher Vernetzung der Masse beträgt 2 Minuten. Die fertige Masse enthält 20 ppm Platin, berechnet als Element.

Beispiel 2

Die in Beispiel 1 beschriebene Arbeitsweise wird wiederholt mit der Abänderung, daß anstelle der 2 g des Gemisches aus Platinkomplex und Verdünnungsmittel, dessen Herstellung oben unter a) beschrieben wurde, 2 g des Gemisches aus Platinkomplex und Verdünnungsmittel, dessen Herstellung oben unter b) beschrieben wurde, eingesetzt werden. Die Zeit zwischen dem Beginn des Vermischens von Platinkomplex enthaltendem, 5 Tage gelagertem Organopolysiloxan mit Si-gebundenen Wasserstoff enthaltendem Organopolysiloxan und merklicher Vernetzung der Masse beträgt 45 Sekunden.

Vergleichsversuch a)

Die in Beispiel 1 beschriebene Arbeitsweise wird wiederholt mit der Abänderung, daß 0,2 g einer 1 % Platin, berechnet als Element, enthaltenden Lösung eines Platin-Divinyltetramethyldisiloxan-Komplexes in Vinyldimethylsiloxygruppen als endständige Einheiten aufweisendem Dimethylpolysiloxan anstelle der 2 g des Gemisches aus Platinkomplex und Verdünnungsmittel, dessen Herstellung oben unter a) beschrieben wurde, eingesetzt werden. Diese Lösung wurde in fast völliger Übereinstimmung mit der eingangs genannten US-PS 38 14 730, Beispiel 6, hergestellt wie folgt:

Zu einer Mischung aus 10 Teilen $H_2PtCl_6 \cdot 6H_2O$, 20 Teilen 1,3-Divinyl-1,1,3,3-tetramethyldisiloxan und 50 Teilen Äthanol wurden 20 Teile Natriumbicarbonat gegeben. Das Gemisch wurde 30 Minuten unter Rühren zum Sieden unter Rückfluß erwärmt, dann 15 Stunden stehengelassen und filtriert. Aus dem Filtrat wurden bei etwa 16 mbar die flüchtigen Bestandteile abdestilliert. Der Rückstand wurde in Benzol gelöst. Die Lösung wurde filtriert und aus dem Filtrat das Benzol abdestilliert. Der Rückstand

wurde in Vinyldimethylsiloxygruppen als endständige Einheiten aufweisendem Dimethylpolysiloxan mit einer Viskosität von 1 000 mPa.s bei 23 °C in solcher Menge gelöst, daß die Lösung 1 % Platin, berechnet als Element, enthält.

Die Zeit zwischen dem Beginn des Vermischens von Platinkomplex enthaltendem, 5 Tage gelagertem Organopolysiloxan mit Si-gebundenen Wasserstoff enthaltendem Organopolysiloxan und merklicher Vernetzung der Masse beträgt 2,5 Minuten.

Beispiel 3

Mischung A: Ein Gemisch aus 102,5 Teilen eines Vinyldimethylsiloxygruppen als endständige Einheiten aufweisenden Dimethylpolysiloxans mit einer Viskosität von 22 000 mPa.s bei 23 °C, 145 Teilen Cristobalitmehl und 5,5 Teilen eines handelsüblichen, pyrogen in der Gasphase erzeugten Siliciumdioxyds mit einer BET-Oberfläche von 200 $m^2$/g, die zu 60 % (ihrer Fläche) durch Behandlung mit Dimethyldichlorsilan hydrophobiert ist, wird mit 10 Teilen des Gemisches aus Platinkomplex und Verdünnungsmittel, dessen Herstellung oben unter a) beschrieben wurde, vermischt, so daß eine Mischung vorliegt, die 40 ppm Platin, berechnet als Element, enthält.

Mischung B: 97 Teile des Vinyldimethylsiloxygruppen als endständige Einheiten aufweisenden Dimethylpolysiloxans mit einer Viskosität von 22 000 mPa.s bei 23 °C werden mit 137 Teilen Cristobalitmehl, 9,2 Teilen des im Zusammenhang mit der Beschreibung der Herstellung der Mischung A näher definierten, teilweise hydrophobierten Siliciumdioxyds und 9,8 Teilen eines Mischpolymerisats aus Dimethylsiloxan-, Methylhydrogensiloxan- und Trimethylsiloxaneinheiten, wobei das Molverhältnis der Dimethylsiloxaneinheiten zu den Methylhydrogensiloxaneinheiten 6:1 beträgt und das Mischpolymerisat eine Viskosität von 200 mPa.s bei 23 °C hat, vermischt.

Die Mischungen A und B werden bei Raumtemperatur 3 Tage gelagert und dann im Gewichtsverhältnis 1:1 miteinander vermischt. Die Zeit, die bei Raumtemperatur zwischen Beginn des Einmischens von Mischung B in Mischung A und merklicher Vernetzung der Masse verstreicht, beträgt 50 Sekunden.

Beispiel 4

Die in Beispiel 3 beschriebene Arbeitsweise wird wiederholt mit der Abänderung, daß anstelle der 10 Teile des Gemisches aus Platinkomplex und Verdünnungsmittel, dessen Herstellung oben unter a) beschrieben wurde, 10 Teile des Gemisches aus Platinkomplex und Verdünnungsmittel, dessen Herstellung oben unter b) beschrieben wurde, eingesetzt werden.

Die Zeit, die bei Raumtemperatur zwischen Beginn des Einmischens von Mischung B in Mischung A und merklicher Vernetzung der Masse verstreicht, beträgt 30 Sekunden.

**Ansprüche**

1. Verfahren zum Anlagern von Si-gebundenem Wasserstoff an aliphatische Mehrfachbindung in Gegenwart von Platinkatalysator, dadurch gekennzeichnet, daß als Platinkatalysator mindestens ein Platinkomplex aus der Gruppe von Verbindungen der allgemeinen Formel

$$(\text{Dien})\ PtX_2,$$

wobei Dien einen cyclischen Kohlenwasserstoff mit 2 aliphatischen Kohlenstoff-Kohlenstoff-Doppelbindungen und X gleiche oder verschiedene Halogenatome und/oder gleiche oder verschiedene Alkylreste bedeutet, und Verbindungen der allgemeinen Formel

$$(R_2SO)\ (Z)\ PtY_2,$$

wobei R gleiche oder verschiedene, gegebenenfalls substituierte Kohlenwasserstoffreste, Z einen Kohlenwasserstoff mit einer Kohlenstoff-Kohlenstoff-Doppelbindung und Y gleiche oder verschiedene Halogenatome bedeutet, verwendet wird.

2. Verfahren nach Anspruch 1, dadurch gekennzeichnet, daß ein Platinkomplex der allgemeinen Formel

$$(\text{Dien})\ PtX_2$$

verwendet wird, worin Dien Dicyclopentadien und X Chlor oder einen Alkylrest mit 1 bis 4 Kohlenstoffatomen bedeutet.

3. Verfahren nach Anspruch 1, dadurch gekennzeichnet, daß als Platinkomplex der allgemeinen Formel

$$(R_2SO)\ (Z)\ PtY_2$$

Dimethylsulfoxyd-äthylen-platin-(II)-dichlorid verwendet wird.

**Claims**

1. Process for the addition of Si-bonded hydrogen to an aliphatic multiple bond in the presence of a platinum catalyst, characterised in that there is used as the platinum catalyst at least one platinum complex from the group of compounds of the general formula

$$(\text{diene})\ PtX_2$$

in which diene denotes a cyclic hydrocarbon having 2 aliphatic carbon-carbon double bonds, and X denotes the same or different halogen atoms and/or the same or different alkyl radicals, and compounds of the general formula

$$(R_2SO)\ (Z)\ PtY_2,$$

in which R denotes the same or different, optionally substituted, hydrocarbon radicals, Z denotes

a hydrocarbon having a carbon-carbon double bond, and Y denotes the same or different halogen atoms.

2. Process according to Claim 1, characterised in that a platinum complex of the general formula

(diene) $PtX_2$

in which diene denotes dicyclopentadiene and X denotes chlorine or an alkyl radical having from 1 to 4 carbon atoms, is used.

3. Process according to Claim 1, characterised in that (dimethyl sulphoxide)ethyleneplatinum(II) dichloride is used as the platinum complex of the general formula

$(R_2SO)$ (Z) $PtY_2$.

**Revendications**

1. Procédé pour fixer de l'hydrogène relié à Si sur une liaison multiple aliphatique, en présence d'un catalyseur au platine, procédé caractérisé en ce qu'on utilise, comme catalyseur au platine, au moins un complexe du platine pris dans l'ensemble constitué par :

— les composés répondant à la formule générale

(diène) $PtX_2$

dans laquelle « diène » représente un hydrocarbure cyclique contenant deux doubles liaisons carbone-carbone aliphatiques et les X représentent des atomes d'halogènes identiques ou différents et/ou des radicaux alkyles identiques ou différents,

et

— les composés répondant à la formule générale

$(R_2SO)$ (Z) $PtY_2$

dans laquelle les R représentent des radicaux hydrocarbonés éventuellement substitués, identiques ou différents, Z représente un hydrocarbure contenant une double liaison carbone-carbone et les Y représentent des atomes d'halogènes identiques ou différents.

2. Procédé selon la revendication 1, caractérisé en ce qu'on utilise un complexe du platine répondant à la formule générale

(diène) $PtX_2$

dans laquelle « diène » représente le dicyclopentadiène et X le chlore ou un radical alkyle contenant de 1 à 4 atomes de carbone.

3. Procédé selon la revendication 1, caractérisé en ce qu'on utilise, comme complexe du platine de formule générale $(R_2SO)$ (Z) $PtY_2$, le dichlorure de diméthylsulfoxyde-éthylène-platine (II).